(19) Europäisches Patentamt
European Patent Office
Office européen des brevets

(11) Veröffentlichungsnummer: **0 170 822**
**B1**

(12) # EUROPÄISCHE PATENTSCHRIFT

(45) Veröffentlichungstag der Patentschrift: **30.08.89**

(51) Int. Cl.[4]: **C 07 D327/06**, C 09 K 15/00

(21) Anmeldenummer: **85107111.8**

(22) Anmeldetag: **10.06.85**

(54) **2,3-Dihydrobenz-4-oxa-1-thiine.**

(30) Priorität: **14.06.84 DE 3421977**

(43) Veröffentlichungstag der Anmeldung: **12.02.86 Patentblatt 86/07**

(45) Bekanntmachung des Hinweises auf die Patenterteilung: **30.08.89 Patentblatt 89/35**

(84) Benannte Vertragsstaaten: **BE CH DE FR GB IT LI NL**

(56) Entgegenhaltungen:
**EP–A– 0 036 169**
**DE–B– 1 167 851**
**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Lechtken, Peter, Dr.**
**Ludwigshafener Strasse 6b**
**D-6710 Frankenthal (DE)**
Erfinder: **Trauth, Hubert, Dipl.-Ing.**
**Milanstrasse 5**
**D-6724 Dudenhofen (DE)**
Erfinder: **Weiss, Stefan, Dr.**
**Carl-Beck-Strasse 46**
**D-6903 Neckargemuend (DE)**
Erfinder: **Hettche, Albert, Dr.**
**Kleiststrasse 12**
**D-6717 Hessheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft 2,3-Dihydrobenz-4-oxa-1-thiine der allgemeinen Formel I

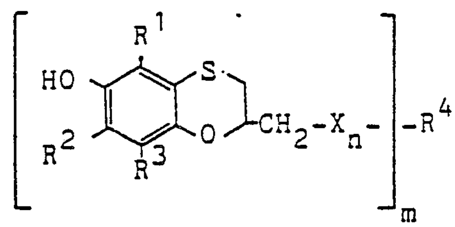

(I)

in welcher die Substituenten und Indices folgende Bedeutung haben :

$R^1$-$R^3$ Wasserstoff, Methyl, Methoxy oder, im Falle von $R^2$ und $R^3$, gemeinsam einen annellierten Benzolring ;

$R^4$ Wasserstoff, einen m-wertigen aliphatischen Kohlenwasserstoffrest, der durch Schwefel unterbrochen sein und/oder Thiolgruppen als Substituenten tragen kann, oder, falls n gleich null ist, Halogen ;

X —O—, —S— oder —O—CO— ;

m 1 oder 2 ;

n null oder 1.

Außerdem betrifft die Erfindung die Herstellungen der Verbindungen I und deren Verwendung als Antioxidantien für organische Materialien sowie organische Materialien, welche die Verbindungen I enthalten.

Verbindungen des Typs I'

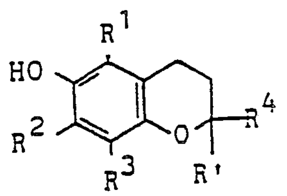

(I')

in welcher $R^1$ bis $R^4$ die oben genannte Bedeutung haben und R' für H oder einen organischen Rest steht, also solche, welche die Struktureinheit des Chromans

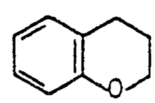

enthalten, sind allgemein als Antioxidantien bekannt. Beispielsweise leitet sich das alpha-Tocopherol (Vitamin E) vom Chroman ab und diverse Chromanderivate sind u.a. in den EP-A 0 036 160, EP-A-0 036 169 und EP-A-0 057 427 beschrieben.

Entsprechend der Aufgabe, die Technik um Antioxidantien zu bereichern, die noch wirksamer sind als die bisher bekannten Chromanderivate I', wurden die eingangs definierten Dihydrobenzoxathiine gefunden.

Die erfindungsgemäßen Verbindungen I unterscheiden sich von den bekannten Chromanderivaten prinzipiell lediglich durch ein Schwefelatom anstelle einer $—CH_2$-Gruppierung in 4-Stellung des Chromangerüstes, sind aber in ihrer Wirksamkeit als Antioxidantien den entsprechenden Chromanverbindungen überraschenderweise überlegen.

Die Verbindungen I sind nach an sich bekannten Methoden erhältlich, wobei der wesentliche Syntheseschritt die Ringschlußkondensation eines Chinons II

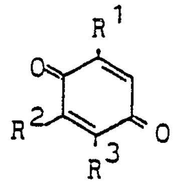

(II)

mit einem Thioglycol III

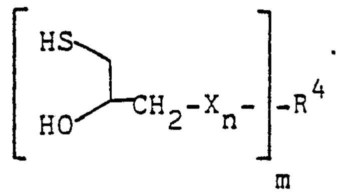

(III)

ist.

Diese Ringschlußkondensation findet bei 40°-160 °C, vorzugsweise 50-110 °C, vorzugsweise in Gegenwart einer Säure, unter gleichzeitiger Wasserabspaltung leicht statt. Als Säuren eignen sich insbesondere Mineralsäuren wie Salzsäure und Phosphorsäure, wobei deren Konzentration zweckmäßigerweise so bemessen wird, daß das Reaktionsgemisch einen pH-Wert aufweist, der unter 1 liegt.

Im allgemeinen empfiehlt es sich, die Umsetzung von II und III in Gegenwart eines Lösungs- oder Verdünnungsmittels vorzunehmen, wobei Wasser bevorzugt wird. Weiterhin kommen wasserlösliche Lösungsmittel wie Alkohole, Tetrahydrofuran und Dioxan sowie deren Mischungen mit Wasser in Betracht. Auch nicht wassermischbare Lösungsmittel wie Toluol, Petrolether und Chlorbenzol können verwendet werden.

Zur Synthese derjenigen Verbindungen I, in denen $R^4$ einen der definitionsgemäßen Kohlenwasserstoffreste bedeutet, kann man auf die vorgenannte Verfahrensweise zunächst auch eine der übrigen Verbindungen I mit m = 1 herstellen, also eine solche mit der Seitenkettengruppierung

$—CH_2—OH$

$—CH_2—SH$ oder

$—CH_2—Hal$ (Hal = Halogen, insbesondere Cl und Br),

die dann anschließend in an sich bekannter Weise mit einer Verbindung IV

$$R^4Y_m \qquad \text{IV}$$

in welcher Y eine funktionelle Gruppe bedeutet, die zur definitionsgemäßen Verknüpfung des Kohlenwasserstoffrestes $R^4$ mit der Gruppierung ($—CH_2—X_n—$) befähigt ist, umgesetzt wird.

Sollen beispielsweise Verbindungen I mit Ether- oder Thioethergruppierung hergestellt werden, setzt man eine Verbindung I mit der Seitenkettengruppierung ($—CH_2—OH$) bzw. ($—CH_2—SH$) beispielsweise mit einer Halogenverbindung IV (Y = Halogen) oder einer olefinischen Verbindung IV (Y = [$CH_2 = CH—$]) um, oder man läßt eine Verbindung I mit der Seitenkettengruppierung $—CH_2—Hal$ umgekehrt mit einem Alkohol bzw. Thiol IV (Y = [HO—] bzw. [HS—]) reagieren.

Verbindungen I mit der Estergruppierung $—CH_2—O—CO—$ können z. B. durch Veresterung oder Umesterung (Y = [—COOH], [—CO Hal], [$—CO—O—CH_3$]) hergestellt werden.

Verlaufen die Verknüpfungsreaktionen unter Abspaltung von Säuren, z. B. von Halogenwasserstoffsäure, so empfiehlt es sich, diese mittels einer Base wie Natronlauge, Pyridin oder Trimethylamin zu binden, und handelt es sich um eine der übrigen Reaktionen wie die Addition an eine olefinische Gruppe oder um die Veresterung eines Alkohols mit einer Säure, so ist die Gegenwart einer starken Säure wie p-Toluolsulfonsäure oder eines sauren Ionenaustauschers zweckmäßig.

Im übrigen gehören die genannten Verknüpfungsreaktionen zu den Standardoperationen der präparativen Chemie, so daß nähere Erläuterungen hierzu entbehrlich sind.

Bedeutet die Gruppierung ($—X_n—R^4$) Halogen, kann man auch die entsprechende Hydroxyverbindung halogenieren.

Die Ausgangsverbindungen II sind bekannt oder in bekannter Weise zugänglich (s. z. B. Houben-Weyl, « Methoden der Organischen Chemie, 4. Auflage, Band 7/3a, Seiten 23-596. Das gleiche gilt für die Thioglycole III (Houben-Weyl, 4. Auflage, Band 9, Seiten 22-23) und die mono- bzw. bifunktionellen Verbindungen IV.

Da die Verbindungen I naturgemäß oxidationsempfindlich sind, ist es zweckmäßig, alle Operationen unter Schutzgasatmosphäre, beispielsweise unter Stickstoff, vorzunehmen.

Under den Verbindungen I werden diejenigen bevorzugt, in denen $R^1$-$R^3$ Methyl bedeuten, die einen aliphatischen $C_2$- bis $C_{18}$-Kohlenwasserstoffrest als Rest $R^4$ im Molekül enthalten und in denen n für 1 steht. Da weiterhin gefunden wurde, daß Schwefel in Thioether- und Thiolfunktion den antioxidativen Effekt verstärkt, werden die definitionsgemäßen schwefelhaltigen Kohlenwasserstoffe als Reste $R^4$ bevorzugt. Solche Reste sind beispielsweise $—CH_2—SH$, $—CH_2—CH_2—S—CH_2—CH_2—$ und $—CH(SH)—CH_3$.

Die erfindungsgemäßen Verbindungen I eignen sich hervorragend als Antioxidantien zur Stabilisierung von organischen Materialien gegen oxidative und thermisch-oxidative Einflüsse. Solche Materialien

sind beispielsweise Kunststoffe, Lebens- und Futtermittel und besonders empfindliche Stoffe wie Farbstoffe, Vitamine, ungesättigte Fette sowie pharmazeutische, veterinärmedizinische und kosmetische Wirkstoffe und Zubereitungen.

Die Konzentration von I in den organischen Materialien liegt allgemein zwischen 0,005 unf 50 Gew.%. Im Falle von Kunststoffen, Lebens- und Futtermitteln reichen Konzentrationen zwischen 0,005 und 1 Gew.% normalerweise aus, wogegen sie im Falle von Vitaminen und ähnlichen hochempfindlichen Stoffen bis zu 50 Gew.% betragen können.

Bei Kunststoffen, vor allem bei Polypropylen, bewirken die Verbindungen I nicht nur eine deutliche Erhöhung der Alterungsbeständigkeit sondern auch der Verarbeitungsstabilität.

Der antioxidative Effekt der Verbindungen I wird, wie weiterhin gefunden wurde, von aliphatischen und cycloaliphatischen Polyolen als Synergisten verstärkt. Solche Polyole, die mindestens 2, vorzugsweise 3-6 freie alkoholische Hydroxylgruppen im Molekül enthalten sollen, sind beispielsweise Ethylenglykol, Glycerin und Trimethylolpropan sowie vor allem Zucker und hydrierte Zucker wie Sorbit. Die Hydroxylgruppen der Polyole können auch z. T. mit Alkanolen verethert oder mit Fettsäuren verestert sein.

Die Konzentration der Polyole in den organischen Materialien liegt zwischen 0,005 und 90 Gew.%, wobei die Summe der Konzentrationen von I und den Polyolen bis zu 99 Gew.% betragen kann. Im Falle weniger empfindlicher Materialien wie Kunststoffen und Lebens- und Futtermitteln beträgt die Konzentration von I etwa 0,001-0,3 Gew.%, wobei das Verhältnis der Konzentration von I und der Polyole vorzugsweise zwischen 1 :1 und 1 :10 liegt.

Die Verbindungen I bereichern ferner die Synthesemöglichkeiten zur Herstelung tocopherolähnlicher Substanzen.

## Beispiele

Im folgenden werden der Rest

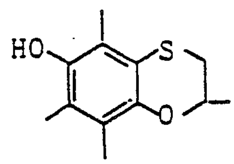

als Rest A und der Rest

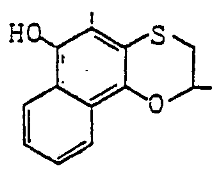

als rest B bezeichnet.

## Beispiel 1

Herstellung von A—$CH_2$—OH

Eine Lösung aus 151 g (1,4 mol) Propan-1,2-diol-3-thiol, 1 l Wasser und 25 ml konzentrierter Salzsäure wurde unter Stickstoffatmosphäre bei 50 °C mit 150 g (1,0 mol) geschmolzenem Trimethylchinon versetzt und 2,5 h zum Sieden erhitzt. Beim anschließenden Abkühlen fiel die oben gennante Verbindung in Form beigeweißer Kristalle an.

Die Kristalle wurden abgetrennt, mit warmem und kaltem Wasser gewaschen, über Calciumchlorid getrocknet und aus 500 ml Acetonitril umkristallisiert; Ausbeute 177 g. Aus der Mutterlauge wurden weitere 46 g des Produktes gewonnen, so daß die Gesamtausbeute 92 % betrug; Smp. 118-120 °C.

## Beispiel 2

Herstellung von A—$CH_2$—Br

48 g (0,2 mol) des Verfahrensproduktes von Beispiel 1 wurden portionsweise zu einem Bromierungsreagens gegeben, welches aus 55 g (0,21 mol) Triphenylphosphin, 1 l trockenem Methylenchlorid und 34 g (0,212 mol) Brom bereitet worden war. Die dunkelbraune Lösung wurde 3 h auf Siedetemperatur erwärmt (ca. 40 °C) erwärmt und sodann in 1 l einer 5 Gew.%igen Natriumhydrogencarbonatlösung eingerührt.

Danach wurde die organische Phase abgetrennt, mit verdünnter NaCl-Lösung gewaschen, mit

$Na_2SO_4$ getrocknet und im Vakuum-Rotationsverdampfer eingedampft. Der Rückstand wurde mit 250 ml Diethylether aufgenommen, wobei sich Triphenylphosphinoxid abschied. Die verbleibende organische Phase wurde eingeengt und mit 200 ml eines Gemisches aus 3 Vol.-Teilen Cyclohexan und 1 Vol.-Teil Ethylacetat aufgenommen, über Kieselgel filtriert und mit ca. 2 l desselben Lösungsmittelgemisches nachgewaschen. Beim Einengen des Filtrates fiel die obengennante Bromverbindung in Form bräunlicher Kristalle aus, die nach Auskochen mit Petrolether einen Smp. von 87-88 °C hatten ; Ausbeute 75 %.

Beispiel 3

Herstellung von B—$CH_2$—OH

Eine Lösung aus 30,2 g (0,28 mol) Propan-1,2-diol-3-thiol, 200 ml Wasser und 5 ml konzentrierter Salzsäure wurde bei 50 °C unter Stickstoffatmosphäre portionsweise mit 34,4 g (0,2 mol) gepulvertem 2-Methyl-1,4-naphthochinon versetzt und 2 h zum Sieden erhitzt. Danach wurde die wäßrige Phase bei 70 °C abgetrennt und die verbleibende Schmelze zweimal bei dieser Temperatur mit dem gleichen Volumen Wasser gewaschen. Beim anschließenden Suspendieren in kaltem Wasser fiel das Produkt als hellgraue Kristallmsse an, die abgetrennt und nach dem Trocknen über $CaCl_2$ aus 200 ml Acetonitril umkristallisiert wurde. Hellgraue bis farblose Kristalle, Smp. 142-144 °C, Ausbeute rd. 70 %.

Beispiel 4

Herstellung von A—$CH_3$

Dieser Verbindung wurde auf die im Beispiel 1 beschriebene Weise aus Trimethylchinon und Propane-2-ol-1-thiol in 60 %iger Ausbeute hergestellt ; Smp. 86-87 °C.

Beispiel 5

Herstellung von (A—$CH_2$—O—CO—$CH_2$—$CH_2$—$)_2$S

24 g (0,1 mol) des Verfahrensproduktes von Beispiel 1 wurden unter Stickstoffatmosphäre zusammen mit 8,9 g (0,05 mol) Thiodipropionsäure und 0,5 g p-Toluolsulfonsäure in 50 ml Toluol erhitzt, wobei das Wasser ausgekreist wurde. Nach 3 bis 4 h wurde das Gemisch abkühlt, wobei ein Teil des Produktes ausfiel. Däs Gemisch wurde mit Methylenchlorid wieder in Lösung gebracht, mit verdünnter $NaHCO_3$-Lösung ausgeschüttelt, mit Wasser gewaschen, getrocknet, mit 2 g Aktivkohle behandelt und im Vakuum-Rotationsverdampfer eigeengt. Der Rückstand wurde aus ca. 50 ml Isopropanol umkristallisiert ; Ausbeute 62 % ; Smp. 81-83 °C.

Beispiel 6

Herstellung von A—$CH_2$—O—CO—$CH_2$—SH

24 g (0,1 mol). des Verfahrensproduktes von Beispiel 1 wurden unter Stickstoffatmosphäre zusammen mit 10,1 g (0,11 mol) Thioglykolsäure und 0,5 g p-Toluolsulfonsäure in 50 ml Toluol unter Entfernung des Wassers erhitzt. Die übliche Aufarbeitung des Reaktionsgemisches lieferte die oben gennante Verbindung in Form eines hellbraunen Öls ; Ausbeute 95 %.

Beispiel 7

Herstellung von A—$CH_2$—O—CO—$CH_{17}H_{35}$

Diese Verbindung wurde analog Beispiel 6 aus der Verbindung A—$CH_2$—OH und Stearinsäure in 61 %iger Ausbeute hergestellt ; Smp. 69-71 °C.

Beispiel 8

Herstellung von B—$CH_2$—O—CO—$C_{17}H_{35}$

Diese Verbindung wurde analog Beispiel 3 aus der Verbindung B—$CH_2$—OH und Stearinsäure in 77 %iger Ausbeute hergestellt, beige Kristalle, Smp. 54-56 °C.

Beispiel 9

Stabilisierende Wirkung verschiedener Antioxidantien in Schweineschmalz

Schweineschmalz mit je einem Gehalt von 0,02 Gew.% verschiedener Antioxidantien wurde dem sogenannten Peroxid-Test (Oil and Soap, Band 15, 1938, S. 184) unterworfen, Hierbei wurden die Proben solange unter Luftzutritt bei 80 °C gelagert, bis sie eine Peroxidzahl (POZ) von 50 hatten. Je länger diese Zeit t dauert, desto wirksamer ist das Antioxidans.

Die Versuchsergebnisse, die für sich selbst sprechen, sind in der Tabelle 1 zu entnehmen.

Tabelle 1

Peroxid-Test bei Schweineschmalz gemäß Beispiel 9

| Versuch | Antioxidans | gemäß Beispiel | t [Tage] |
|---|---|---|---|
| Zum Vergleich | | | |
| 1 V | alpha-Tocopherol | - | 1 |
| 2 V | 2,5,7,8-Tetramethyl-2-(2-hydroxy-eth-1-yl)-6-hydroxychroman | - | 5 |
| 3 V | wie 2 V, jedoch in der Seitenkette mit Stearinsäure verestert | - | 4 |
| erfindungsgemäß | | | |
| 1 | $A-CH_2-OH$ | 1 | 7 |
| 2 | $B-CH_2-OH$ | 3 | 12 |
| 3 | $(A-CH_2-O-CO-CH_2CH_2-)_2S$ | 5 | 13 |
| 4 | $A-CH_2-O-CO-CH_2-SH$ | 6 | 10 |
| 5 | $B-CH_2-O-CO-C_{17}H_{35}$ | 8 | 9 |

Beispiel 10

Stabilisierende Wirkung verschiedener Antioxidantien in Canthaxanthin-Trockenpulver

Canthaxanthin, das als Zusatz zu Legehennenfutter dient, wurde mit 4 Gew.% des Antioxidans $A—CH_2—O—CO—C_{17}H_{35}$ gemäß Beispiel 7 vermischt und mit Gelatine wie üblich zu einem Trockenpulver verarbeitet. Dieses Pulver wurde sodann in einer Konzentration von 200 ppm Wirkstoff mit handelsüblichem Legehennenfutter vermischt und 8 Wochen bei 40 °C und 70 % relativen Luftfeuchtigkeit gelagert, wonach der Canthaxanthin-Gehalt noch 66 % der ursprünglichen Konzentration betrug.

In einer Vergleichsprobe ohne Zusatz eines Antioxidans war kein Canthaxanthin mehr nachweisbar, und bei Verwendung des Antioxidans gemäß Versuch 3 V in Tabelle 1 betrug der Wirkstoffgehalt nur noch 43 %.

Beispiel 11

Stabilisierung von Polypropylen-Verarbeitungsstabilisierung

Reines Polypropylenpulver des mittleren Molekulargewichtes 10 000 wurde mit jeweils 0,1 Gew.% eines Antioxidans bzw. einer Mischung verschiedener Antioxidantien vermischt und bei 250 °C extrudiert und zu Granulat zerkleinert. Extrusion und Granulierung wurden mehrfach wiederholt, wobei nach dem 1., 3., 5. und 8. dieser Vorgänge der Schmelzindex MFI der jeweiligen Granulatprobe bei 190 °C nach DIN 53 135 gemessen wurde. Je höher der Schmelzindex, desto größer ist der Abbau des Polypropylens infolge der thermischen Belastung bei der Extrusion und desto geringer ist die Verarbeitungsstabilität.

Außerdem wurde die Färbung festgestellt, welche das Material nach der 8. Extrusion auswies.

Die Ergebnisse der Versuche sind der Tabelle 2 zu entnehmen.

(Siehe Tabelle 2 Seite 10 f.)

Beispiel 12

Stabilisierung von Polypropylen : Langzeitstabilität

Das Material gemäß Beispiel 11 wurde nach DIN 53 383 (Ofenalterung) bei 220 °C zunächst zu Granulat und sodann bei 220 °C zu Platten von 20 × 20 × 1 $mm^3$ verarbeitet, die in einem Umluftwärmeschrank mit Frischluftzufuhr bei 140 °C gelagert wurden.

Hierbei versprödeten die Platten, wobei jeweils die Zeit t festgestellt wurde, die einem bestimmten, für alle Versuche gleichen Versprödungsgrad entsprach. Diese Zeit ist nicht nur ein unmittelbares Maß für die Ofenalterung sondern indirekt auch für die Langzeitstabilität.

Tabelle 2

Stabilisierung von Polypropylen gemäß den Beispielen 11 und 12

| Versuch Nr. | Antioxidans gemäß Beispiel | Synergist | q 1) | Farbe 2) | MFI-Werte nach Passagen 1 | 3 | 5 | 8 | Ofenalterung t [h] |
|---|---|---|---|---|---|---|---|---|---|
| Zum Vergleich | | | | | | | | | |
| 1 V | - | Sorbit | - | 0 | 7 | 13 | 20 | 30 | 20 |
| 2 V | s.3V aus Tabelle 1 | - | 0 | 3 | 4 | 6 | 7 | 8 | 200 |
| 3 V | " | Sorbit | 2 | 3 | 4 | 5 | 6 | 7 | 200 |
| 4 V | " | " | 4 | 2 | 5 | 6 | 9 | 11 | 150 |
| erfindungsgemäß | | | | | | | | | |
| 1 | Beispiel 7 | - | 0 | 3 | 5 | 6 | 8 | 10 | 400 |
| 2 | " | Sorbit | 2 | 3 | 5 | 6 | 7 | 9 | 290 |
| 3 | " | " | 4 | 2 | 5 | 5 | 6 | 7 | 200 |
| 4 | " | " | 6 | 2 | 5 | 6 | 7 | 10 | - 3) |

1) Mengenverhältnis Synergist/Antioxidans.
2) Zwischen 0 (farblos) und 6 (braun).
3) Nicht gemessen.

Die Ergebnisse dieser Versuche sind ebenfalls der Tabelle 2 zu entnehmen.

## Patentansprüche

1. 2,3-Dihydrobenz-4-oxa-1-thiine der allgemeinen Formel I

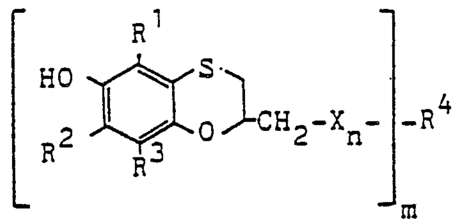

(I)

in welcher die Substituenten und Indices folgende Bedeutung haben :

$R^1$-$R^3$ Wasserstoff, Methyl, Methoxy oder, im Falle von $R^2$ und $R^3$, gemeinsam einen annellierten Benzolring ;

$R^4$ Wasserstoff, einen m-wertigen aliphatischen Kohlenwasserstoffrest, der durch Schwefel unterbrochen sein und/oder Thiolgruppen als Substituenten tragen kann, oder, falls n gleich null ist, Halogen ;

X —O—, —S— oder —O—CO— ;

m 1 oder 2 ;

n null oder 1.

2. Verfahren zur Herstellung der Verbindungen I, dadurch gekennzeichnet, daß man ein Chinon der allgemeinen Formel

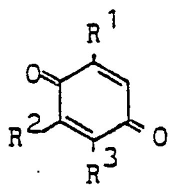

(II)

mit einem Thioglycol der allgemeinen Formel III

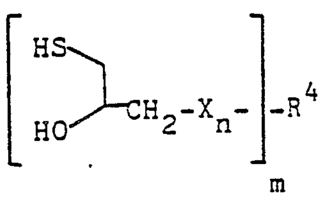

(III)

umsetzt.

3. Verfahren zur Herstellung von Verbindungen I, in denen $R^4$ einen der definitionsgemäßen Kohlenwasserstoffreste beudetet, dadurch gekennzeichnet, daß man zunächst gemäß Anspruch 2 eine der Verbindungen I mit m = 1 und der Seitenkettengruppierung

$—CH_2—OH$

$—CH_2—SH$ oder

$—CH_2—Hal$ (Hal = Halogen, insbesondere Cl und Br

herstellt und diese in an sich bekannter Weise mit einer Verbindung IV

$$R^4Y_m \qquad IV$$

umsetzt, in der Y eines funktionelle Gruppe ist, die zur definitionsgemäßen Verknüpfung des Kohlenwasserstoffrestes $R^4$ mit der Gruppierung ($—CH_2—X_n—$) befähigt ist.

4. Verwendung der Verbindungen I als Antioxidanten für oxidationsempfindliche organische Materialien.

5. Gegen oxidative und thermisch-oxidative Einflüsse stabilisierte organische Materialien, gekennzeichnet durch einen Gehalt von 0,005 bis 50 Gew.% einer der Verbindungen I.

6. Gegen oxidative und thermisch-oxidative Einflüsse stabilisierte organische Materialien gemäß Anspruch 5, gekennzeichnet durch einen zusätzlichen Gehalt von 0,005 bis 90 Gew.% eines aliphatischen oder cycloaliphatischen Polyols.

## Claims

1. A 2,3-dihydrobenz-4-oxa-1-thiin of the formula I

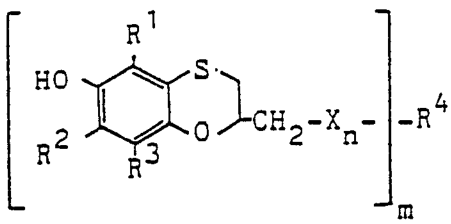

(I)

where

$R^1$, $R^2$ and $R^3$ are each hydrogen, methyl or methoxy, and $R^2$ and $R^3$ together may furthermore be a fused benzene ring,

$R^4$ is hydrogen or an m-valent aliphatic hydrocarbon radical which may be interrupted by sulfur and/or carry thiol groups as substituents, or is halogen when n is zero,

X is —O—, —S— or —O—CO—,

m is 1 or 2 and

n is zero or 1.

2. A process for the preparation of a compound I, wherein a quinone of the formula

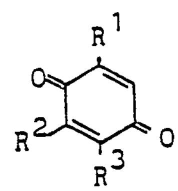

(II)

is reacted with a thiolglycol of the formula III

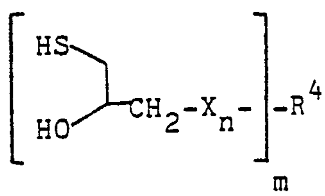

(III)

3. A process for the preparation of a compound I in which $R^4$ is one of the hydrocarbon radicals of the type defined, wherein one of the compounds I in which m is 1 and which contains the side chain group

$—CH_2—OH$,

$—CH_2—SH$ or

$CH_2—Hal$ (where Hal is halogen, in particular Cl or Br), is first prepared according to claim 2, and this compound is reacted in a conventional manner with a compound IV

$$R^4Y_m \qquad IV$$

where Y is a functional group capable of linking the hydrocarbon radical $R^4$ to the group $—CH_2—X_n—$ in conformity with the definition.

4. Use of a compound I as an antioxidant for organic materials which are sensitive to oxidation.

5. An organic material which is stable to oxidation and thermal oxidation, containing from 0.005 to 50 % by weight of one of the compounds I.

6. An organic material which is stable to oxidation and thermal oxidation, as claimed in claim 5, additionally containing from 0.005 to 90 % by weight of an aliphatic or cycloaliphatic polyol.

## Revendications

1. 2,3-dihydrobenz-4-oxa-1-thiine de la formule générale I

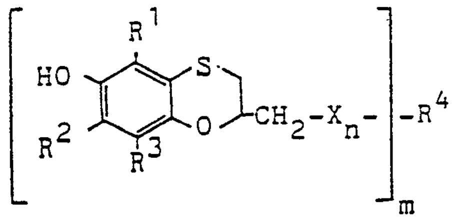

(I)

dans laquelle les substituants et indices ont les significations suivantes :

$R^1$-$R^3$ représentent hydrogène, méthyle, méthoxy ou dans les cas de $R^2$ et $R^3$, ensemble un noyau benzène condensé ;

$R^4$ représente hydrogène, un reste d'hydrocarbure aliphatique de valeur m, qui peut être interrompu par du soufre, et/ou peut porter des groupes thiol comme substituants, ou, dans le cas où $n = 0$, représente halogène ;

X représente —O—, —S— ou —O—CO— ;

m est 1 ou 2 ;

n est nul ou 1.

2. Procédé de préparation des composés I, caractérisé par le fait qu'on fait réagir une quinone de formule générale

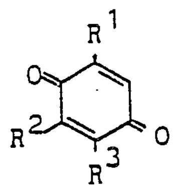

(II)

avec un thioglycol de formule générale III

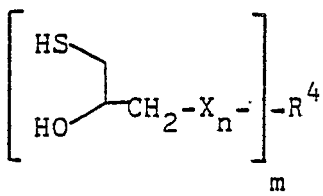

(III)

3. Procédé de préparation de composés I, dans lesquels $R^4$ représente un des restes d'hydrocarbure selon la définition, caractérisé par le fait que l'on prépare d'abord selon la revendication 2 un des composés I avec $m = 1$ et les groupements de chaîne latéraux

$—CH_2—OH$,

$—CH_2—SH$ ou

$—CH_2—Hal$ (Hal = halogène en particulier Cl et Br), et on fait réagir celui-ci avec un composé IV

$$R^4Y_m \qquad IV$$

dans lequel Y est un groupe fonctionnel qui est apte à la liaison, selon la définition, du reste d'hydrocarbure $R^4$ avec le groupement ($—CH_2—X_n—$).

4. Utilisation des composés I comme antioxydants pour les matériaux organiques sensibles à l'oxydation.

5. Matériaux organiques stabilisés vis-à-vis des influences oxydantes et thermooxydantes, caractérisés par le fait qu'il contiennent 0,005 à 50 % en poids d'un des composés I.

6. Matériaux organiques stabilisés vis-à-vis des influences oxydantes et thermooxydantes selon la revendication 5, caractérisés par une teneur additionnelle de 0,005 à 90 % en poids en un polyol alyphatique ou cycloaliphatique.